(19) Europäisches Patentamt
European Patent Office
Office européen des brevets

(11) **EP 4 549 887 A1**

(12) **EUROPEAN PATENT APPLICATION**

(43) Date of publication:
**07.05.2025 Bulletin 2025/19**

(21) Application number: **24203883.4**

(22) Date of filing: **01.10.2024**

(51) International Patent Classification (IPC):
**G01F 1/68** (2006.01)     **G01N 25/16** (2006.01)
**G01N 25/66** (2006.01)     **G01F 1/688** (2006.01)

(52) Cooperative Patent Classification (CPC):
**G01F 1/6888; G01N 25/16; G01N 25/66;**
G01N 27/18; H10N 10/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.10.2023 IN 202311073755**

(71) Applicant: Honeywell International Inc.
**Charlotte, NC 28202 (US)**

(72) Inventors:
• KUMAR, Nirmal A.
  Charlotte, 28202 (US)
• MCCOLLUM, David R.
  Charlotte, 28202 (US)
• PRATT, Keith Francis Edwin
  Charlotte, 28202 (US)
• SHANBHOGUE, Shridhara
  Charlotte, 28202 (US)

(74) Representative: Haseltine Lake Kempner LLP
Cheapside House
138 Cheapside
London EC2V 6BJ (GB)

(54) **THERMAL CONDUCTIVITY DETECTOR BASED GAS SENSOR AND ASSOCIATED METHOD**

(57) A sensing device is provided. For example, a sensing device may include a conducting element, a DC voltage source, a voltage measuring device, a first switching circuitry to selectively connect the DC voltage source to the conducting element, and a processor that controls the first switching circuitry. The conducting element comprises first and second dissimilar materials arranged such that there is a first junction between the dissimilar materials and a second junction between the dissimilar materials. The processor controls the first switching circuitry to connect the DC voltage source to the conducting element to apply a DC voltage for a first period of time and to disconnect the DC voltage source to measure a voltage over a second period of time.

**FIG. 1**

EP 4 549 887 A1

**Description**

FIELD OF THE INVENTION

Embodiments of the present disclosure generally relate to sensing devices, and, more particularly, to thermal conductivity detector (TCD) based sensing devices.

BACKGROUND

Heated sensing devices, such as thermal conductivity gas sensing devices, raise the sensing element to a desired operating temperature. In many such devices, there is a heating element that is separate from the sensing element to raise the sensing element to the desired operating temperature.

Such heated sensing devices are plagued by technical challenges and limitations. Through applied effort, ingenuity, and innovation, many of these identified problems have been solved by developing solutions that are included in embodiments of the present disclosure, many examples of which are described in detail herein.

BRIEF SUMMARY

Various embodiments described herein relate to sensing devices and associated methods of sensing thermally conductive gas and/or air flow.

In accordance with various embodiments of the present disclosure, a sensing device is provided. In some embodiments, the sensing device comprises a substrate, a conducting element positioned on the substrate and comprising first and second dissimilar materials arranged such that there is a first junction between the first and second dissimilar materials and a second junction between the first and second dissimilar materials, a direct current (DC) voltage source, a voltage measuring device, a first switching circuitry for selectively connecting the DC voltage source to the first and second connection terminals of the conducting element, and a processor that controls the first switching circuitry. The conducting element further comprises first and second connection terminals. The processor controls the first switching circuitry to selectively connect the DC voltage source to the first and second connection terminals of the conducting element to apply a DC voltage across the first and second connection terminals of the conducting element for a first period of time and to selectively disconnect the DC voltage source from the first and second connection terminals of the conducting element to measure a voltage across the first and second connection terminals over a second period of time.

In some embodiments, the first and second dissimilar materials of the conducting element are arranged such that there is a plurality of first junctions between the first and second dissimilar materials and a plurality of second junctions between the first and second dissimilar materials.

In some embodiments, the substrate comprises a first portion and a second portion that is thinner than the first portion, the plurality of first junctions is positioned on the first portion of the substrate, and the plurality of second junctions is positioned on the second portion of the substrate.

In some embodiments, the first period of time is a time period which is long enough for the voltage across the first and second connection terminals to reach a steady state voltage and the second period of time is a time period which is long enough for a temperature of the first junction and a temperature of the second junction to decrease by a measurable amount.

In some embodiments, the processor determines the steady state voltage across the first and second connection terminals and, using the determined steady state voltage, determines (i) a presence and/or a concentration of a thermally conductive gas adjacent the conductive element and/or (ii) a presence and/or an amount of air flow across the conductive element.

In some embodiments, the processor determines a time constant of the voltage across the first and second connection terminals over the second period of time and, using the determined time constant, determines (i) a presence and/or a concentration of a thermally conductive gas adjacent the conductive element and/or (ii) a presence and/or an amount of air flow across the conductive element.

In some embodiments, the first and second dissimilar materials comprise first and second dissimilar conductive or semi-conductive materials.

In some embodiments, the first and second dissimilar materials comprise, respectively, chromel and alumel.

In some embodiments, the processor causes the DC voltage source to apply a plurality of different voltages across the first and second connection terminals of the conducting element, each different voltage applied a first time with a first polarity and a second time with a second, opposite polarity, the processor causes the voltage measuring device to measure a voltage across the first and second connection terminals of the conducting element after each of the plurality of different voltages are applied, and the processor detects when an abrupt change occurs in a time constant of the voltage across the first and second connection terminals and determines a temperature that coincides with the abrupt change in the time constant of the voltage across the first and second connection terminals, the temperature being a dewpoint.

In some embodiments, the DC voltage source comprises one or more batteries.

In accordance with various embodiments of the present disclosure, a method of sensing thermally conductive gas and/or air flow is provided. In some embodiments, the method comprises causing, by a processor of a sensing device, a first switching circuitry to selectively connect a direct current (DC) voltage source to first and second connection terminals of a conducting element; applying, by the DC voltage source, a DC voltage across the first and second connection terminals of the conducting element for a first period of time; causing, by the processor of the sensing device, the first switching circuitry to selectively disconnect the DC voltage source from the first and second connection terminals of the conducting element; and measuring, by the voltage measuring device, a voltage across the first and second connection terminals over a second period of time. The conducting element comprises first and second dissimilar materials arranged such that there is a first junction between the first and second dissimilar materials and a second junction between the first and second dissimilar materials.

The foregoing illustrative summary, as well as other exemplary objectives and/or advantages of the disclosure, and the manner in which the same are accomplished, are further explained in the following detailed description and its accompanying drawings.

BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:

 FIG. 1 is a block diagram of an example sensing device in accordance with example embodiments of the present disclosure;
 FIG. 2 is a model of an example sensing device in accordance with example embodiments of the present disclosure;
 FIG. 3 is a model of an example sense element of an example sensing device in accordance with alternative example embodiments of the present disclosure;
 FIG. 4 is a graph illustrating the voltage response of a sensing element of an example sensing device in accordance with example embodiments of the present disclosure; and
 FIG. 5 is a flowchart illustrating an example method of operating an example sensing device in accordance with example embodiments of the present disclosure.

DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. In the present disclosure, the term "element" may refer to an article, a device, or an apparatus that may provide one or more functionalities.

The term "sensing device" may refer to an article, a device, or an apparatus that measures physical input from its environment and converts the input into data that can be interpreted by either a human or a machine. Sensing devices may be utilized in a variety of applications including the detection of dangerous gases in the environment. Sensing devices may be mounted in a fixed location or may be portable so as to be carried by a user. Portable sensing devices are typically battery-powered. One common type of sensing device is a thermal conductivity gas sensing device which can measure the concentration of gas according to the difference of thermal conductivity of different gases and air.

The term "sensor," "sensor circuitry," "sensing circuitry," "sensing element," "sense element," and the like may refer to one or more components, integrated circuits, and the like within a sensing device that interacts with the environment, detects the input to be detected (e.g., presence of a dangerous gas), and provides an output (e.g., voltage) to another component in the sensing device to be interpreted. In some conventional thermal conductivity gas sensing devices, the sensing circuitry comprises a resistive heating element and one or more thermopiles. When power is applied to the resistive heating element, a temperature gradient is produced across the thermopile(s) which produces an output voltage that corresponds to the temperature difference. When exposed to a gas with higher thermal conductivity than air (e.g., hydrogen), there is more heat loss from the resistive heating element and therefore a lower temperature. This difference in temperature varies with the concentration of the gas and can be detected, measured, and used to provide an alert.

To address challenges and limitations associated with conventional sensing devices, various examples of the present disclosure may be provided. For example, various examples of the present disclosure may provide example sensing devices, apparatuses, methods, and systems having a sense element without a separate heating element as compared to some conventional sensing devices. An example sensing device includes a sense element that makes use of the Peltier Effect and the Seebeck Effect as described herein. The Peltier Effect provides that a voltage applied across a thermocouple causes a temperature difference between the junctions of the different materials in the thermocouple. The Seebeck Effect provides that a temperature difference between two dissimilar electrical conductors or semiconductors produces a voltage difference between the two materials.

Various embodiments of the present disclosure provide an example sensing device having a sense element comprising first and second dissimilar conductive or semi-conductive materials arranged such that there is at least a first junction between first and second dissimilar materials and at least a second junction between first and second dissimilar materials. In various embodiments, a direct current (DC) voltage is applied across terminals of the sense element for a first predetermined period of time, which, according to the Peltier Effect, causes one or more of the junctions to become hotter than ambient air and one or more of the junctions to become colder than ambient air. In various embodiments, when the DC voltage is removed from the terminals of the sense element, junctions are at different temperatures and, according to the Seebeck effect, a voltage is generated across the terminals of the sense element due to the temperature differential. In various embodiments, the voltage across the terminals of the sense element is measured over a second predetermined time period. In various embodiments, which of the junctions become hotter and which become cooler can be varied by reversing the polarity of the applied DC voltage.

In various embodiments, after the DC voltage is removed from the terminals of the sense element the temperature difference between the junctions will decrease by a measurable amount. In various embodiments, after the DC voltage is removed from the terminals of the sense element the temperature of each of the junctions will reach the ambient air temperature.

In various embodiments, the DC voltage is applied across terminals of the sense element by closing a first switch assembly and the DC voltage is removed from the terminals of the sense element by opening the first switch assembly. In various embodiments, a processor controls the closing and opening of the first switch assembly.

In various embodiments, the rate of heat dissipated from the junctions to surrounding air will increase in the presence of gases with a higher thermal conductivity than air (hydrogen or helium) and/or with an increase in the flow of air across the sense element. In such conditions, the time for the junction temperatures to reach equilibrium state will be reduced. Generally, the time to reach thermal equilibrium decreases about one percent for every one percent increase in the concentration of hydrogen in the air.

In various embodiments, the shape and characteristics of the measured voltage signal changes as the temperature difference dissipates. In various embodiments, the shape and characteristics of the measured voltage signal can be correlated to the presence and/or concentration of gases with a higher thermal conductivity than air and/or to the presence and amount of air flow across the sense element.

In various embodiments, a voltage measuring device (e.g., a voltmeter) is selectively connected to the sense element to measure the voltage across the terminals of the sense element. In various embodiments, the voltage measuring device is connected to the sense element by closing a second switch assembly and the voltage measuring device is disconnected from the sense element by opening the second switch assembly. In various embodiments, a processor controls the closing and opening of the second switch assembly.

In various embodiments, the sense element is mounted on a substrate. In various embodiments, the sense element comprises a plurality of thermocouples forming a thermopile, such that there is a plurality of first junctions between the first and second dissimilar materials and a plurality of second junctions between the first and second dissimilar materials. In various embodiments, the substrate comprises a thinner portion and a thicker portion, such that the plurality of first junctions is positioned on the thinner portion of the substrate and the plurality of second junctions is positioned on the thicker portion of the substrate.

Embodiments of the present disclosure may be used with any suitable sensing device, including but not limited to gas detection sensors, air flow sensors, and dewpoint/humidity sensors.

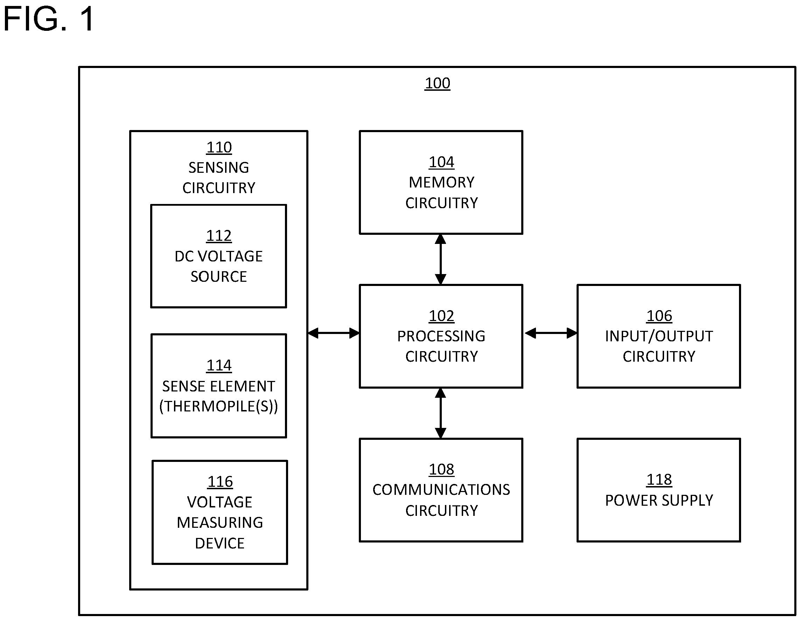

Referring now to FIG. 1, a block diagram of an example sensing device 100 in accordance with various embodiments of the present disclosure is provided. As depicted in FIG. 1, in some embodiments the sensing device 100 comprises a processor or processing circuitry 102, memory circuitry 104, input/output circuitry 106, communications circuitry 108, sensing circuitry 110, and a power supply 118. In some embodiments, the sensing device 100 is configured to execute and perform the operations described herein.

Although components are described with respect to functional limitations, it should be understood that at least some of the particular implementations necessarily include the use of particular computing hardware. It should also be understood that in some embodiments certain of the components described herein include similar or common hardware. For example, in some embodiments two sets of circuitry both leverage use of the same processor(s), memory(ies), circuitry(ies), and/or the like to perform their associated functions such that duplicate hardware is not required for each set of circuitry.

Processing circuitry 102 may be embodied in a number of different ways. In various embodiments, the use of the terms "processor" or "processing circuitry" should be understood to include a single core processor, a multi-core processor, multiple processors internal to the sensing device 100, and/or one or more remote or "cloud" processor(s) external to the sensing device 100. In some example embodiments, processing circuitry 102 may include one or more processing devices configured to perform independently. Alternatively, or additionally, processing circuitry 102 may include one or more processor(s) configured in tandem via a bus to enable independent execution of operations, instructions, pipelining, and/or multithreading.

In an example embodiment, the processing circuitry 102 may be configured to execute instructions stored in the memory circuitry 104 or otherwise accessible to the processor. Alternatively, or additionally, the processing circuitry 102 may be configured to execute hard-coded functionality. As such, whether configured by hardware or software methods, or by a combination thereof, processing circuitry 102 may represent an entity (e.g., physically embodied in circuitry) capable of performing operations according to embodiments of the present disclosure while configured accordingly. Alternatively, or additionally, processing circuitry 102 may be embodied as an executor of software instructions, and the instructions may specifically configure the processing circuitry 102 to perform the various algorithms embodied in one or more operations described herein when such instructions are executed. In some embodiments, the processing circuitry 102 includes hardware, software, firmware, and/or a combination thereof that performs one or more operations described herein.

In some embodiments, the processing circuitry 102 (and/or co-processor or any other processing circuitry assisting or otherwise associated with the processor) is/are in communication with the memory circuitry 104 via a bus for passing information among components of the sensing device 100.

Memory or memory circuitry 104 may be non-transitory and may include, for example, one or more volatile and/or non-volatile memories. In some embodiments, the memory circuitry 104 includes or embodies an electronic storage device (e.g., a computer readable storage medium). In some embodiments, the memory circuitry 104 is configured to store information, data, content, applications, instructions, or the like, for enabling the sensing device 100 to carry out various operations and/or functions in accordance with example embodiments of the present disclosure.

Input/output circuitry 106 may be included in the sensing device 100. In some embodiments, input/output circuitry 106 may provide output to the user and/or receive input from a user. The input/output circuitry 106 may be in communication with the processing circuitry 102 to provide such functionality. The input/output circuitry 106 may comprise one or more user interface(s). In some embodiments, a user interface may include a display that comprises the interface(s) rendered as a web user interface, an application user interface, a user device, a backend system, or the like. In some embodiments, the input/output circuitry 106 also includes a keyboard, a mouse, a joystick, a touch screen, touch areas, soft keys, a microphone, a speaker, or other input/output mechanisms. The processing circuitry 102 and/or input/output circuitry 106 may be configured to control one or more operations and/or functions of one or more user interface elements through computer program instructions (e.g., software and/or firmware) stored on a memory accessible to the processor (e.g., memory circuitry 104, and/or the like). In some embodiments, the input/output circuitry 106 includes or utilizes a user-facing application to provide input/output functionality to a computing device and/or other display associated with a user.

Communications circuitry 108 may be included in the sensing device 100. The communications circuitry 108 may include any means such as a device or circuitry embodied in either hardware or a combination of hardware and software that is configured to receive and/or transmit data from/to a network and/or any other device, circuitry, or module in communication with the sensing device 100. In some embodiments the communications circuitry 108 includes, for example, a network interface for enabling communications with a wired or wireless communications network. Additionally or alternatively, the communications circuitry 108 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 108 may include circuitry for interacting with an antenna(s) and/or other hardware or software to cause transmission of signals via the antenna(s) and/or to handle receipt of signals received via the antenna(s). In some embodiments, the communications circuitry 108 enables transmission to and/or receipt of data from a user device, one or more sensors, and/or other external computing device(s) in communication with the sensing device 100.

The sensing circuitry 110 may comprise any suitable circuitry to provide the desired sensing functionality. In the case of a thermal conductivity gas sensing device, the sensing circuitry 110 comprises a DC voltage source 112, a sense element 114 (comprising one or more thermopiles), and a voltage measuring device 116.

The power supply 118 provides electrical power to the sensing device 100, including power to the sensing circuitry 110. The power supply 118 may comprise any suitable power supply, and, for a portable sensing device is likely to comprise one or more batteries.

In some embodiments, two or more of the sets of circuitries 102-110 are combinable. Alternatively, or additionally, one or more of the sets of circuitry 102-110 perform some or all of the operations and/or functionality described herein as being associated with another circuitry. In some embodiments, two or more of the sets of circuitry 102-110 are combined into a single module embodied in hardware, software, firmware, and/or a combination thereof.

While the description above provides an example sensing device 100, it is noted that the scope of the present disclosure is not limited to the description above. In some examples, an example sensing device 100 in accordance with the present disclosure may be in other forms. In some examples, an example sensing device 100 may comprise one or more additional and/or alternative elements, and/or may be structured differently than that illustrated in FIG. 1.

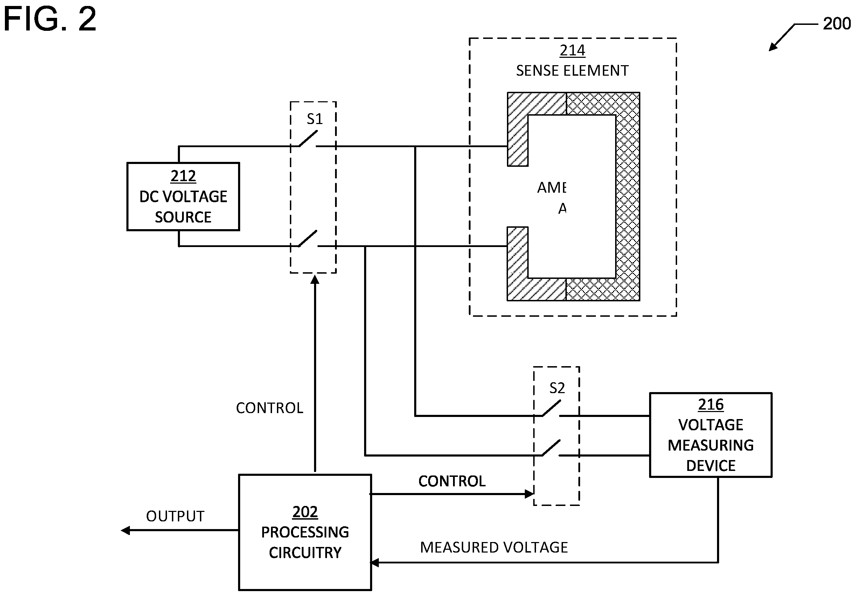

Reference will now be made FIG. 2, which is a model of an example sensing device in accordance with example embodiments of the present disclosure. The example sensing device 200 of FIG. 2 comprises a processing circuitry 202, a DC voltage source 212, a sense element (e.g., thermocouple) 214, a voltage measuring device 216, a first switch assembly S 1, and a second switch assembly S2. Similar to as described above, the DC voltage source 212 is selectively connected to the sense element 214 via the first switch assembly S 1, and the voltage measuring device 216 (e.g., a voltmeter) is selectively connected to the sense element 214 via the second switch assembly S2. In the simplified model of FIG. 2, the sense element 214 comprises a single thermocouple having two dissimilar materials (one material indicated by hatching and the other material is indicated by cross-hatching). The two dissimilar materials are arranged such that there are two junctions J1, J2 between the two dissimilar materials. In various embodiments, the thermocouple of the sense element 214 is exposed to ambient air for the sensing device 200 to be able to detect a gas with higher thermal conductivity than air based on the rate of heat dissipated from the junctions J1, J2 to the surrounding air. Because the rate of heat dissipated from the junctions J1, J2 also increases with an increase in the flow of air across the sense element, in some embodiments the sense element 214 in a device to be used as a gas sensor will be positioned in a housing that has a structure (e.g., baffles) that permits ambient air to reach the sense element 214 but shields the sense element 214 from flowing air. Conversely, embodiments the sense element 214 in a device to be used as a flow sensor will be positioned in a housing that has a structure (e.g., a tunnel) that permits flowing air to reach the sense element 214.

In the illustrated embodiment of FIG. 2, the processing circuitry 202 sends control signals to the first switch assembly S 1 and the second switch assembly S2 to control the closing and opening of the switches thereof. The processing circuitry 202 causes the first switch assembly S1 to close and the second switch assembly S2 to open to connect the DC voltage source 212 to the sense element 214 and to disconnect the voltage measuring device 216 from the sense element 214. Because of the two dissimilar materials, the voltage applied across the sense element 214 will cause the temperature of one junction (e.g., J1) to rise above the ambient temperature and the temperature of the other junction (e.g., J2) to decrease below the ambient temperature due to the Peltier Effect. Which junction sees a temperature increase and which sees a temperature decrease depends on the polarity of the applied DC voltage and can be changed by changing the polarity of the applied DC voltage.

The processing circuitry 202 causes the second switch assembly S2 to close and the first switch assembly S2 to open to connect the voltage measuring device 216 to the sense element 214 and to disconnect the DC voltage source 212 from the sense element 214. After the DC voltage is disconnected from the sense element 214, the temperatures of the two junctions will begin to equilibrate and, over time, return to the ambient temperature. Because of the two dissimilar materials, the temperature difference between two dissimilar electrical conductors or semiconductors produces a voltage difference between the two materials due to the Seebeck Effect. That voltage will decrease over time as the temperature difference decreases. The voltage measuring device 216 measures this voltage over a time period as the temperature difference decreases and the voltage decreases correspondingly. Thus, in some embodiments, the same sense element (i.e., same thermocouple(s)) is used both to produce the temperature differential and then to measure the voltage produced by the temperature differential as the temperature differential decreases. In this regard, a separate heating element is not needed in various embodiments of the invention.

In some embodiments, the first and second dissimilar materials of the sense element are conductive or semi-conductive materials. For example, the first and second dissimilar materials may comprise, respectively, chromel and alumel. Chromel is an alloy of nickel and chrome plus nine other elements. Alumel is an alloy containing nickel manganese, aluminum, silicon and nine other elements. However, any suitable dissimilar conductive or semi-conductive materials may be used. For example, in various embodiments, any of the materials listed in FIG. 11 of U.S. Patent No. 11,747,184, titled THERMOPILE-BASED FLOW SENSING DEVICE, issued September 5, 2023, may be used as the first and/or second dissimilar materials described herein. The contents of U.S. Patent No. 11,747,184 are incorporated herein in its entirety.

In the illustrated embodiment of FIG. 2, the processing circuitry 202 is connected to the voltage measuring device 216 to receive the measured voltage. In some embodiments, the processing circuitry 202 processes the measured voltage (as described further below) to determine a presence and/or a concentration of a thermally conductive gas adjacent the sense element 214 and/or a presence and/or an amount of air flow across the sense element 214. In some embodiments, the processing circuitry 202 produces an output signal to send the determined gas concentration and/or air flow to, for example, a display element and/or a central monitoring device for display to a user.

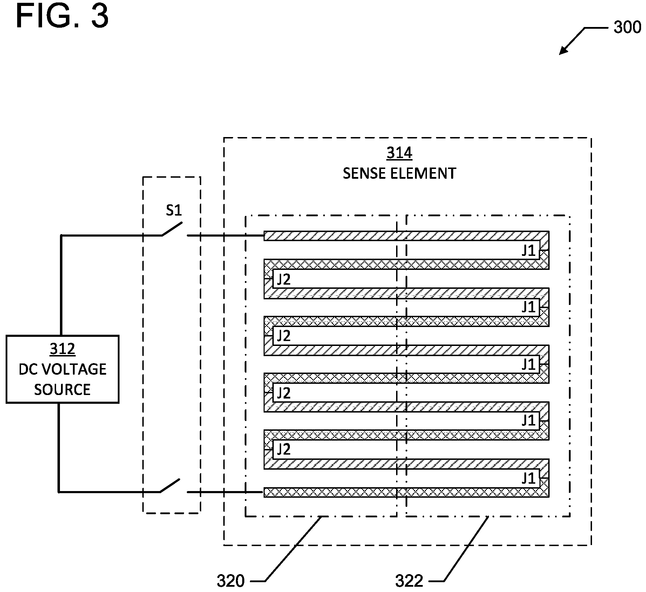

Reference will now be made FIG. 3, which is a model of an example sense element of an example sensing device in accordance with example embodiments of the present disclosure. The example sensing device 300 of FIG. 3 comprises a DC voltage source 312, a sense element 314, and a first switch assembly S1. For simplicity, FIG. 3 omits a voltage measuring device, a processing circuitry, and a second switch assembly. In the embodiment illustrated in FIG. 3, the sense element 314 comprises multiple thermocouples wired in series, such that there are multiple segments of the two dissimilar materials (one material indicated by hatching and the other material is indicated by cross-hatching), with multiple first junctions J1 (five are shown) and multiple second junctions J2 (four are shown). Having multiple thermocouples wired in series causes a greater temperature differential when a voltage is applied by the DC voltage source 312 and a greater voltage produced by the temperature difference between dissimilar materials, thereby providing a voltage that is easier to measure.

In various embodiments, the sense element is mounted on a (typically planar) substrate, such as a silicon substrate. In various embodiments, a thinner substrate is preferred so that the substrate itself doesn't conduct too much heat away from the sense element and the main source of heat loss is thermal conductivity through the gas that is surrounding the sense element rather than thermal conductivity through the substrate.

In some embodiments, the substrate has a thinner portion and a thicker portion. In the illustrated embodiment of FIG. 3, the substrate includes a thinner portion 320 upon which the second junctions J2 are mounted and a thicker portion 322 upon which the first junctions J1 are mounted.

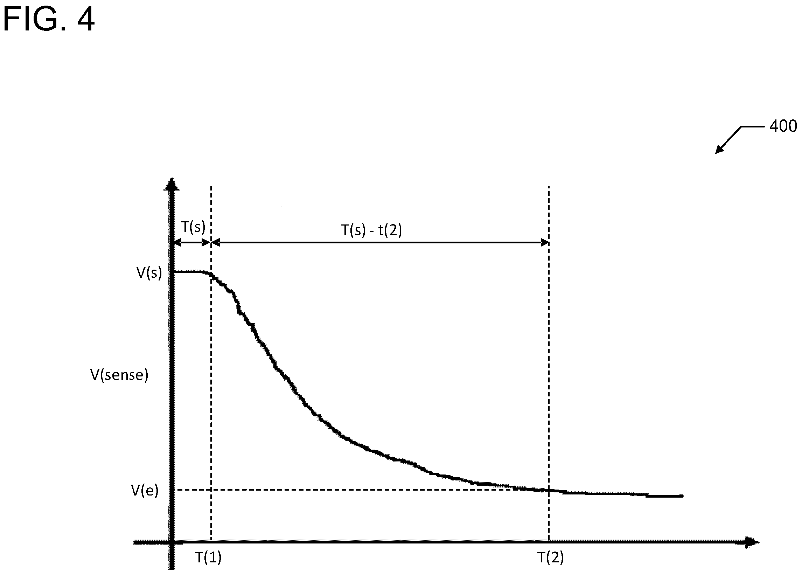

As described above, when a DC voltage is applied to the terminals of a sense element of a thermal conductivity gas sensing device as described herein, a temperature gradient is produced across the thermopile(s) which produces an output voltage that corresponds to the temperature. Reference will now be made FIG. 4, which is a graph 400 illustrating the voltage response of an example thermal conductivity gas sensing device. Graph 400 of FIG. 4 shows the voltage (V(sense), y-axis) across the terminals of an example sense element of an example sensing device over time (t, x-axis). In graph 400, time t(s) is the time after the DC voltage has been applied and the temperature differential (and therefore the voltage) has reached its maximum (this may be termed the "steady state time"), time t(1) is when the DC voltage has been removed from the terminals of an example sense element and (after a short delay) the temperature and the corresponding voltage first begin to change, time t(2) is the end of the measurement time period, V(e) is the voltage across the sense element at time t(2), and V(s) is the initial or steady state voltage (during time t(s)). The end t(2) of the measurement time period may be, for example, when the steady state voltage V(s) has decreased by 50 percent, when the voltage V(sense) has reached zero (indicating that the temperature of the junctions has equilibrated), or any other suitable time.

Due to the faster temperature dissipation as described above, the presence of a gas with a higher thermal conductivity than air or the presence of air flow will cause the steady state voltage V(s) to be lower and will cause the voltage V(sense) to decrease faster after the voltage is removed from the sense element. As such, the steady state voltage V(s) is proportional to the concentration of a gas with a higher thermal conductivity than air and is proportional to the air flow. Similarly, the time constant ($\tau$) of the voltage V(sense) curve is also proportional to the concentration of a gas with a higher thermal conductivity than air and is proportional to the air flow. Thus, in various embodiments the presence of a gas with a higher thermal conductivity than air and/or the presence of air flow can be determined using the measured steady state voltage V(s) and/or the time constant ($\tau$) of the voltage V(sense) curve.

In some embodiments, the time constant ($\tau$) of the voltage V(sense) curve can be determined using the equation:

$$\frac{1}{\tau} = -\frac{1}{t(2)-t(s)} \ln \left(\frac{V(e)}{V(s)}\right)$$

Once the time constant ($\tau$) is determined, V(sense) at any time (t) can be calculated using the equation:

$$V(\text{sense}) = V(s) * e^{-\frac{t}{\tau}}$$

In some embodiments, the steady state voltage (V(s)) and/or the time constant ($\tau$) when only air is present (i.e., no gas with a higher thermal conductivity than ambient air) and/or when no air flow is present is determined and stored for use in gas and/or air flow detection. Such an advance determination of the steady state voltage (V(s)) and/or the time constant ($\tau$) when only air is present and/or when no air flow is present may be performed in any suitable manner and at any suitable time. For example, in some embodiments the steady state voltage (V(s)) and/or the time constant ($\tau$) when only air is present and/or when no air flow is present is determined during calibration of the device. Such a calibration may occur, for example, during the manufacturing process. In some embodiments, the steady state voltage (V(s)) and/or the time constant ($\tau$) are also determined at multiple different gas concentrations and/or air flows and stored for use in gas and/or air flow detection. In this regard, in some embodiments a measured steady state voltage (V(s)) and/or time constant ($\tau$) is compared to stored values for steady state voltage (V(s)) and/or time constant ($\tau$) to determine the gas concentration and/or air flow.

In some embodiments, a time constant can be determined by measuring how much time it takes for the voltage to decrease by a predetermined amount.

In some embodiments, it is not needed for the voltage during powering to reach a steady state. If it is known how long the DC voltage has been applied and how hot it has gotten, it is still possible to use the device as described herein, albeit with more complicated signal processing. Keeping the powering part of the process as short as possible has the benefit of minimizing power consumption.

In some embodiments, the substrate has a uniform thickness. For example, in some embodiments the substrate may comprise planar silicon that is about 10-15 thousandths of an inch thick. In some embodiments, all of the junctions of the sense element are thermally isolated from the substrate. In some embodiments, some junctions (such as the first junctions J1) of the sense element are thermally isolated from the substrate while other junctions (such as the second junctions J2) are not thermally isolated from the substrate. Embodiments in which all of the junctions of the sense element are thermally isolated from the substrate may be termed "symmetrical," such that applying a voltage to the sense element causes one set of junctions (such as the first junctions J1) to get hotter than the substrate and the other set of junctions (such as the second junctions J2) to get colder. Embodiments in which some junctions of the sense element are thermally isolated from the substrate while other junctions are not thermally isolated from the substrate may be termed "asymmetrical," such that one set of junctions (such as the first junctions J1) remains close to the substrate temperature and the other (floating) set of junctions (such as the second junctions J2) heats or cools relative to the substrate. In some embodiments, the thermally isolated junctions are thermally isolated because the junctions are positioned on a relatively thin substrate.

In some symmetrical embodiments, the polarity of the applied DC voltage is changed with each measurement cycle, such that the hot and cold junctions of the device swap on each alternate measurement cycle and the resulting temperature gradient and hence thermopile output voltage is reversed on each alternate measurement cycle. In such embodiments, offset voltage errors in the electronics may cancel each other out.

For a device to be classed as 'intrinsically safe,' the maximum temperature of the device must be kept below the temperature at which flammable gases can be ignited. This maximum temperature can be as low as 100C depending on the flammable gases present. By having one end of the device heating and the other cooling (symmetrical sensor), double the temperature difference can be generated for the same upper temperature. For the asymmetrical sensor, the floating end could be cooled rather than heated relative to the substrate so that the maximum temperature of the device remains below ambient temperature. In this regard, in some embodiments of the invention excessive temperature rise above gradient may be avoided.

In some asymmetrical embodiments, in dry air the output signal due to thermal conductivity of the air should be the same regardless of whether the floating end is heated or cooled relative to ambient (apart from the reversed polarity). Similarly, the output voltage signal should simply scale with applied voltage and the transient signal should remain constant with applied voltage because the heat loss from the device is the same in all conditions. However, if the floating end is cooled below the dewpoint of the air, there will be an abrupt change in heat loss due to condensation occurring on the floating end, hence a signal that is very different from that measured at other voltages or when the floating end is heated. So, in effect the device of some embodiments of the disclosure can act as both a thermal conductivity device and similarly to a chilled mirror dewpoint meter by doing multiple measurements at different applied voltages and polarities and noting when an abrupt change in heat loss occurs. In some embodiments, the measured dewpoint can be used to calculate the absolute humidity which can then be used to apply compensation to the gas thermal conductivity measurement.

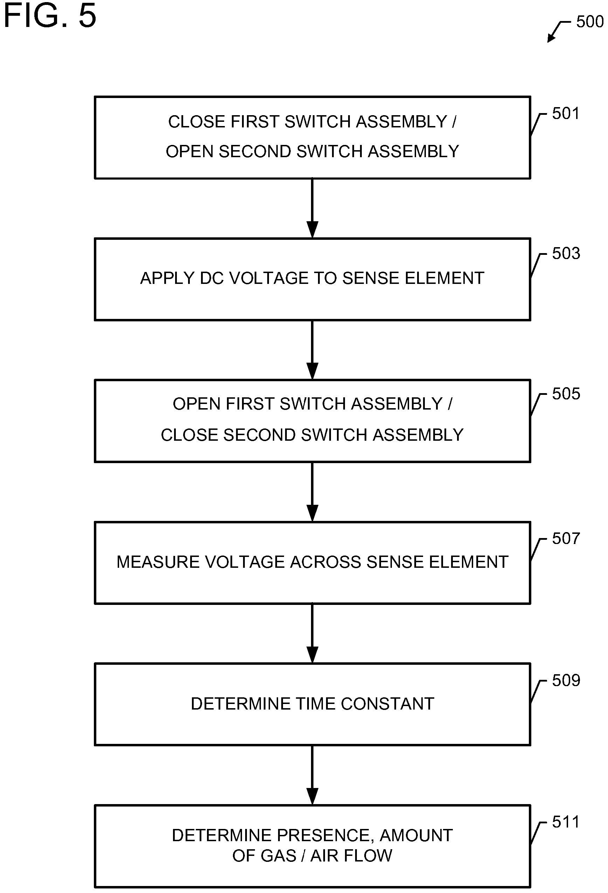

Reference will now be made to FIG. 5, which provides a flowchart illustrating example steps, processes, procedures, and/or operations in accordance with various embodiments of the present disclosure. Various methods described herein, including, for example, methods as shown in FIG. 5, may provide various technical benefits and improvements.

Referring now to FIG. 5, an example method 500 is illustrated. In some embodiments, the example method comprises a method of sensing thermally conductive gas and/or air flow. At step/operation 501, a processor (such as, but not limited to, the processing circuitry 102 of the example sensing device 100 described above in connection with FIG. 1) causes a first switch assembly to close and a second switch assembly to open. As described above, closing such a first switch assembly connects a DC voltage source (such as, but not limited to, the DC voltage source 112 of the example sensing device 100 described above in connection with FIG. 1) to a sense element (such as, but not limited to, the sense element 114 of the example sensing device 100 described above in connection with FIG. 1).

At step/operation 503, a DC voltage source (such as, but not limited to, the DC voltage source 112 of the example sensing device 100 described above in connection with FIG. 1) applies a DC voltage to a sense element. The applied DC voltage may have any suitable magnitude and duration. In some example embodiments, a DC voltage in the range of millivolts to volts is applied for a few tens of milliseconds, depending on how physically big the sense element is (generally, the larger the sense element, the longer the DC voltage must be applied to reach the steady state voltage. This voltage should typically be at a level which can cause a significant temperature difference (e.g., about 100 degrees Celsius) across the junctions.

At step/operation 505, a processor (such as, but not limited to, the processing circuitry 102 of the example sensing device 100 described above in connection with FIG. 1) causes a first switch assembly to open and a second switch assembly to close. As described above, opening such a first switch assembly disconnects the DC voltage source and closing such a second switch assembly connects a voltage measuring device (such as, but not limited to, the voltage measuring device 116 of the example sensing device 100 described above in connection with FIG. 1) to the sense element.

At step/operation 507, a voltage measuring device (such as, but not limited to, the voltage measuring device 116 of the example sensing device 100 described above in connection with FIG. 1) measures the voltage across the sense element. As described above, when the second switch assembly is initially closed, the temperature difference between the junctions will be at its maximum, there will be a short delay and then the temperature difference between the junctions will begin to equilibrate and the measured voltage will start to decay down towards zero.

Although not illustrated in FIG. 5, as described above, the measured voltage right when the second switch assembly is initially closed (i.e., the steady state voltage) can be used to determine the presence and/or concentration of a thermally conductive gas and/or the presence and/or amount of air flow.

At step/operation 509, a processor (such as, but not limited to, the processing circuitry 102 of the example sensing device 100 described above in connection with FIG. 1) determines a time constant of the voltage curve as described above.

At step/operation 511, a processor (such as, but not limited to, the processing circuitry 102 of the example sensing device 100 described above in connection with FIG. 1) uses the determined time constant of the voltage curve to determine the presence and/or concentration of a thermally conductive gas and/or the presence and/or amount of air flow as described above.

In some embodiments, the method repeats steps/operations 501-511 continuously or periodically.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. A sensing device comprising:

   a substrate;
   a conducting element positioned on the substrate, the conducting element comprising first and second dissimilar materials arranged such that there is a first junction between the first and second dissimilar materials and a second junction between the first and second dissimilar materials, the conducting element further comprising first and second connection terminals;
   a direct current (DC) voltage source;
   a voltage measuring device;
   a first switching circuitry for selectively connecting the DC voltage source to the first and second connection terminals of the conducting element; and

a processor that controls the first switching circuitry to selectively connect the DC voltage source to the first and second connection terminals of the conducting element to apply a DC voltage across the first and second connection terminals of the conducting element for a first period of time and to selectively disconnect the DC voltage source from to the first and second connection terminals of the conducting element to measure a voltage across the first and second connection terminals over a second period of time.

2. The sensing device of claim 1, wherein the first and second dissimilar materials of the conducting element are arranged such that there is a plurality of first junctions between the first and second dissimilar materials and a plurality of second junctions between the first and second dissimilar materials.

3. The sensing device of claim 2, wherein the substrate comprises a first portion and a second portion that is thinner than the first portion;

wherein the plurality of first junctions is positioned on the first portion of the substrate; and
wherein the plurality of second junctions is positioned on the second portion of the substrate.

4. The sensing device of claim 1, wherein the first period of time is a time period which is long enough for the voltage across the first and second connection terminals to reach a steady state voltage; and
wherein the second period of time is a time period which is long enough for a temperature of the first junction and a temperature of the second junction to decrease by a measurable amount.

5. The sensing device of claim 4, wherein the processor determines the steady state voltage across the first and second connection terminals; and
wherein the processor, using the determined steady state voltage, determines (i) a presence and/or a concentration of a thermally conductive gas adjacent the conductive element and/or (ii) a presence and/or an amount of air flow across the conductive element.

6. The sensing device of claim 4, wherein the processor determines a time constant of the voltage across the first and second connection terminals over the second period of time; and
wherein the processor, using the determined time constant, determines (i) a presence and/or a concentration of a thermally conductive gas adj acent the conductive element and/or (ii) a presence and/or an amount of air flow across the conductive element.

7. The sensing device of claim 1, wherein the first and second dissimilar materials comprise first and second dissimilar conductive or semi-conductive materials.

8. The sensing device of claim 1, wherein the processor causes the DC voltage source to apply a plurality of different voltages across the first and second connection terminals of the conducting element, each different voltage applied a first time with a first polarity and a second time with a second, opposite polarity;

wherein the processor causes the voltage measuring device to measure a voltage across the first and second connection terminals of the conducting element after each of the plurality of different voltages are applied; and
wherein the processor detects when an abrupt change occurs in a time constant of the voltage across the first and second connection terminals and determines a temperature that coincides with the abrupt change in the time constant of the voltage across the first and second connection terminals, the temperature being a dewpoint.

9. A method of sensing thermally conductive gas and/or air flow, the method comprising:

causing, by a processor of a sensing device, a first switching circuitry to selectively connect a direct current (DC) voltage source to first and second connection terminals of a conducting element, the conducting element comprising first and second dissimilar materials arranged such that there is a first junction between the first and second dissimilar materials and a second junction between the first and second dissimilar materials;
applying, by the DC voltage source, a DC voltage across the first and second connection terminals of the conducting element for a first period of time;
causing, by the processor of the sensing device, the first switching circuitry to selectively disconnect the DC voltage source from the first and second connection terminals of the conducting element; and
measuring, by a voltage measuring device, a voltage across the first and second connection terminals over a second period of time.

10. The method of claim 9, wherein the first and second dissimilar materials of the conducting element are arranged such that there is a plurality of first junctions between the first and second dissimilar materials and a plurality of second junctions between the first and second dissimilar materials.

**11.** The method of claim 10, wherein the plurality of first junctions is positioned on a first portion of a substrate; and

wherein the plurality of second junctions is positioned on a second portion of the substrate that is thinner than the first portion.

**12.** The method of claim 9, wherein the first period of time is a time period which is long enough for the voltage across the first and second connection terminals to reach a steady state voltage; and

wherein the second period of time is a time period which is long enough for a temperature of the first junction and a temperature of the second junction to decrease by a measurable amount.

**13.** The method of claim 12, further comprising:

determining, by the processor of the sensing device, the steady state voltage across the first and second connection terminals; and

determining, by the processor of the sensing device using the determined steady state voltage, (i) a presence and/or a concentration of a thermally conductive gas adjacent the conductive element and/or (ii) a presence and/or an amount of air flow across the conductive element.

**14.** The method of claim 12, further comprising:

determining, by the processor of the sensing device, a time constant of the voltage across the first and second connection terminals over the second period of time; and

determining, by the processor of the sensing device using the determined time constant, (i) a presence and/or a concentration of a thermally conductive gas adjacent the conductive element and/or (ii) a presence and/or an amount of air flow across the conductive element.

**15.** The method of claim 9, further comprising:

applying a plurality of different voltages across the first and second connection terminals of the conducting element, each different voltage applied a first time with a first polarity and a second time with a second, opposite polarity;

measuring a voltage across the first and second connection terminals of the conducting element after each of the plurality of different voltages are applied; and

detecting an abrupt change in a time constant of the voltage across the first and second connection terminals and determining a temperature that coincides with the abrupt change in the time constant of the voltage across the first and second connection terminals, the temperature being a dewpoint.

FIG. 1

FIG. 2

200

214
SENSE ELEMENT

AME
A

212
DC VOLTAGE
SOURCE

S1

216
VOLTAGE
MEASURING
DEVICE

S2

CONTROL

MEASURED VOLTAGE

CONTROL

202
PROCESSING
CIRCUITRY

OUTPUT

# FIG. 3

300

## 314
### SENSE ELEMENT

312
DC VOLTAGE
SOURCE

S1

J1
J2
J1
J2
J1
J2
J1
J2
J1

320          322

FIG. 4

# FIG. 5

500

```
┌──────────────────────────────────────┐
│     CLOSE FIRST SWITCH ASSEMBLY /     │─ 501
│      OPEN SECOND SWITCH ASSEMBLY      │
└──────────────────────────────────────┘
                    │
                    ▼
┌──────────────────────────────────────┐
│    APPLY DC VOLTAGE TO SENSE ELEMENT  │─ 503
└──────────────────────────────────────┘
                    │
                    ▼
┌──────────────────────────────────────┐
│      OPEN FIRST SWITCH ASSEMBLY /     │─ 505
│      CLOSE SECOND SWITCH ASSEMBLY     │
└──────────────────────────────────────┘
                    │
                    ▼
┌──────────────────────────────────────┐
│   MEASURE VOLTAGE ACROSS SENSE ELEMENT│─ 507
└──────────────────────────────────────┘
                    │
                    ▼
┌──────────────────────────────────────┐
│        DETERMINE TIME CONSTANT        │─ 509
└──────────────────────────────────────┘
                    │
                    ▼
┌──────────────────────────────────────┐
│     DETERMINE PRESENCE, AMOUNT        │─ 511
│        OF GAS / AIR FLOW              │
└──────────────────────────────────────┘
```

Europäisches
Patentamt

European
Patent Office

Office européen
des brevets

# EUROPEAN SEARCH REPORT

**Application Number**

EP 24 20 3883

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 677 416 A (NISHIMOTO IKUO [JP] ET AL) 30 June 1987 (1987-06-30) <br> * column 1, lines 4-8; figures 1-7 * <br> * column 3, line 41 - column 4, line 65 * <br> * column 6, lines 53-54 * <br> * column 7, lines 17-26 * <br> * column 4, lines 35-65 * <br> ----- | 1-3,7, 9-11 | INV. <br> G01F1/68 <br> G01N25/16 <br> G01N25/66 <br> G01F1/688 |
| X <br><br> Y | US 4 501 145 A (BOEGLI JEAN-CHARLES [FR] ET AL) 26 February 1985 (1985-02-26) <br> * column 6, lines 37-68; figures 4,5 * <br> * column 3, line 67 - column 4, line 2 * <br> * column 2, line 63 - column 3, line 9 * <br> ----- | 1,4,6,7, 9,12,14 <br> 3,11 |  |
| X <br><br><br><br><br><br><br> Y | MACHUT C ET AL: "NEW PELTIER SENSOR FOR MEASURING THE THERMAL CONDUCTIVITY OF FLUIDS", <br> IEEE TRANSACTIONS ON INSTRUMENTATION AND MEASUREMENT, IEEE, USA, <br> vol. 47, no. 2, 1 April 1998 (1998-04-01), pages 398-402, XP000864857, <br> ISSN: 0018-9456, DOI: 10.1109/19.744181 <br> * Par. II.B.1, II.B.2, IV, "Conclusions" * <br> ----- | 1,2,4,5, 7,9,10, 12,13 <br><br><br><br><br><br> 3,11 | TECHNICAL FIELDS SEARCHED (IPC) <br><br> G01F <br> G01N <br> H10N |
| X | WO 2021/162644 A1 (GORENJE D O O [SI]; HISENSE GUANGDONG KITCHEN & BATH SYS CO LTD [CN]) 19 August 2021 (2021-08-19) <br> * pages 8,9,11; claim 3 * <br> ----- | 1,8,9,15 |  |
| Y | US 2006/220164 A1 (MURTHY SUNIL S [IN] ET AL) 5 October 2006 (2006-10-05) <br> * paragraphs [0024] - [0030]; figures 8-11 * <br> ----- | 3,11 |  |

The present search report has been drawn up for all claims

| Place of search | Date of completion of the search | Examiner |
|---|---|---|
| Munich | 17 February 2025 | De Masi, Rita |

EPO FORM 1503 03.82 (P04C01)

## ANNEX TO THE EUROPEAN SEARCH REPORT
## ON EUROPEAN PATENT APPLICATION NO.

EP 24 20 3883

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2025

| Patent document cited in search report | | Publication date | Patent family member(s) | | Publication date |
|---|---|---|---|---|---|
| US 4677416 | A | 30-06-1987 | JP | H0374945 B2 | 28-11-1991 |
| | | | JP | S61124859 A | 12-06-1986 |
| | | | US | 4677416 A | 30-06-1987 |
| US 4501145 | A | 26-02-1985 | EP | 0070801 A1 | 26-01-1983 |
| | | | JP | S58501094 A | 07-07-1983 |
| | | | US | 4501145 A | 26-02-1985 |
| | | | WO | 8300227 A1 | 20-01-1983 |
| WO 2021162644 | A1 | 19-08-2021 | CN | 115803613 A | 14-03-2023 |
| | | | EP | 4078162 A1 | 26-10-2022 |
| | | | WO | 2021162644 A1 | 19-08-2021 |
| US 2006220164 | A1 | 05-10-2006 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11747184 B